# EUROPEAN PATENT SPECIFICATION

(11) **EP 0 666 271 B1**
(45) Date of publication and mention of the grant of the patent: **20.10.1999**
(21) Application number: 95200257.4
(22) Date of filing: 02.02.1995
(51) Int. Cl.: C07K 14/245, C07K 1/36, A61K 38/16, A61K 39/108, C07K 16/12

(54) **Type 1 and type P fimbriae-adhesins isolated from novel E. coli strains, process for their preparation and uses thereof**
Aus neuen E.Coli Stämmen isolierte Typ 1 und Typ P Fibrillen-Adhesine, Verfahren zu ihrer Herstellung und ihre Verwendungen
Fibrilles-Adhésines de type 1 et de type P isolées des nouvelles souches d'E.coli, procédé pour leur préparation et leur utilisation

(30) Priority: 04.02.1994 ES 9400202
(43) Date of publication of application: 09.08.1995
(73) Proprietor: INDUSTRIAL FARMACEUTICA Y DE ESPECIALIDADES, S.A., E-48008 Bilbao (ES)
(72) Inventor: Palacios Pelaez, Ricardo, Madrid (ES); Martinez Garate, Alberto, Santurce (Vizcaya) (ES); Martinez Quesada, Jorge, Bilbao (ES)
(74) Representative: Ungria Lopez, Javier

(56) References cited:
- EP-A- 0 102 831
- WO-A-90/00614
- DE-A- 3 832 785

## Description

### TECHNICAL FIELD OF THE INVENTION

The present invention is comprised within the technical field of products used to treat and prevent infections of the urinary tract.

More specifically, the present invention provides fimbriae isolated from novel E. coli strains in a highly purified state that have shown to be very effective for the foreseen purposes, in animals. Therefore, said fimbriae are potentially useful to prevent and treat the cited infections in humans.

The cited novel E. coli strains are specifically Escherichia coli CECT 4484 and Escherichia coli CECT 4485 deposited by the applicant in the Spanish Culture Type Collection (Colección Española de Cultivos Tipo - CECT) of the University of Valencia on 19 January 1994, in accordance with the "Budapest Treaty on International Recognition of the Deposit of Microorganisms for the purposes of the Process in Patent Material", both resulting as feasible strains in conditions of "Reseeding in suitable culture media" when deposited.

### PRIOR ART OF THE INVENTION

Escherichia coli is the most frequent etiological agent in infections of the urinary tract, that cause symptoms of acute and chronic cystitis, as well as pyelonephritis and asymptomatic bacteriuria ((1) "Problemática de las infecciones urinarias en España" (Problem of urinary infections in Spain) Liade, 1989; (2) Woodrow GC, Levine MM New generation vaccines, 1990).

It is known that infections of the urinary tract are caused by Escherichia coli strains that contain adhesin structures that are capable of combining with the uroepithelial cells ((3) Johnson JM, Virulence factors in E. coli UTI. Clin. Microbiol. Reviews 1991; 4; 80-128) and that said structures are one of the main causes of pathogenicity of these organisms in the urinary tract (3). These structures are comprised of protein subunits with a tubular shape that contains a terminal and/or side region where it combines with the receptor (3) and (4) Brinton CC. The structure, function, synthesis and genetic control of bacterial pili and molecular model for DNA and RNA transport in gram negative bacteria. Trans NY Acad. Sci. 1965; 27; 1003-1054.) Said structures are named fimbriae and several families that are associated to factors of virulence: mannose resistent fimbriae (type P:P, F or NAP, type X or Dr related: 5, M, 6) and mannose sensitive fimbriae (type 1) have been determined (3.) Type X, AFA-I and AFA-III mannose resistant adhesins are not associated to fimbrial structures. Both types of fimbriae have been tested as preventive and/or protective agents of infection of the urinary tract by uropathogenic E. coli ((5) Hagberg L, Leffler H, Svanborg-Eden C. Non-antibiotic prevention of UTI: Infection 1985; 13 (Suppl. 2): 196-200; (6) O'Hanley P. Lark D, Falkow S, Schoolnik G. Molecular basis of E. coli colonization of the upper urinary tract in Balb/c mice. J. Clin. Invest. 1985; 75: 347-360; (7) Hutlgren SJ, Porter TN, Schaeffer AJ, Duncan JL. Role of type I pili and effects of phase variation on lower UTI produced by E. colo. Infect. Immun. 1985; 50: 370-377; (8) Silverblatt FS, Weinstein R. Rene P. Protection against experimental pyelonephritis by antibodies to pili. Science J. Infect Dis 1982; suppl. 33: 79-82).

The object of the study was to purify and identify fimbriae-adhesins of uropathogenic E. coli isolated from urine samples of women with recurrent cystitis, to use them as a product for preventive treatment of infections of the urinary tract.

There are already studies conducted on the production and effectiveness of E. coli fimbriae used as vaccines to prevent infections of the urinary tract. Thus, aside from the publication of Pecha et al. ((9) Pecha B, Low D. O'Hanley P. Gal-Gal pili vaccines prevent pyelonephritis by piliated E. coli in murine model. J. Clin. Invest. 1989; 83:2102-2108), the following patents may be cited:
- DE-A-3832785 regarding regarding the isolation of p-adhesin from E. coli cells and use of the same for immunization, production of antibodies and diagnosis.
- EP-A-0342173 regarding a new fibronectin bonding protein expressed as fimbria on E. coli and hybrid DNA encoding sequences, to prevent E. coli infections by means of vaccination or topical application.
- EP-A-0314224 regarding a vaccine for the protection of fowl against septicemia by E. coli that contains immunogenic sections or type F11 fimbriae or antibodies against them.
- EP-A-0249739 regarding a vaccine for immunization against infections of the urinary tract that comprise fimbriae with fimbriae of the same fimbric family as the infecting organisms.
- EP-A-0179887 regarding an antigen that comprises a polypeptide adhesin determinant, useful in immunization against bacterial infections and in the production of antibodies for diagnosis of infections.
- EP-A-0161095 regarding vaccines against urinary tract infections containing fragments or proteins of gal-gal fimbriae of E. coli.
- EP-A-0546584 regarding the prepation of vaccines from Escherichia coli with pharmacological uses to prevent or cure gastroenteritis.

In contrast to the above cited prior art, the present invention proposes the preparation and purification of type 1 and type P fimbriae-adhesins, from novel E. coli strains isolated from urine of women with recurrent cystitis, with specific characteristics that will be indicated further on. By means of the present invention it is possible to obtain intact fimbriae-adhesins being free from other proteins or structures such as flagella, hemolysin and LPS that could affect the destination of the product and, additionally, to ensure the elimination of the toxic substances from the product in question.

On the other hand, the practical total absence of crossed reactivity between type 1 fimbriae-adhesins and the type P ones isolated here, allow the manufacture of immunogenes that have each one of the types of fimbriae separately, as well as the two types of fimbria-adhesin at the same concentration and that have shown protective capacity in the studies carried out.

Besides, the verification of the adherence capacity of E. coli strains associated to the fimbriae allows working with a material that induces immunoresponse against the adhesin fraction of the same cause of the fixation of E. coli to uroepithelial cells.

### BRIEF DESCRIPTION OF THE FIGURES

Figure 1 is a microphotograph taken by electronic microscopy techniques, of the purified fimbriae coming from the E. coli strain CECT 4484.

Figure 2 is a microphotograph taken by electronic microscopy techniques, of the purified fimbriae coming from the E. coli strain CECT 4485.

Figure 3 is a graph of the results of the ELISA study Inhibition of uropathogenes with type 1 fimbriae in solid phase.

Figure 4 is a graph of the results of the ELISA study Inhibition of uropathogenes with type P fimbriae in solid phase.

### DETAILED DESCRIPTION OF THE INVENTION

The invention as claimed invention refers to type 1 and type P fimbriae-adhesins isolated from novel E. coli strains, to a process for their preparation and to the uses thereof.

The process of the present invention is based on the already classic one described by Brinton (4), using as the starting material novel strains of uropathogenic E. coli strains isolated from urine of women with recurrent cystitis, wherein it was verified that there were adhesins associated to type 1 and type P fimbriae. Specifically, E. coli strains CECT 4484 and CECT 4485, whose characteristics are indicated in the following table 1, were used:

**TABLE 1**

| Uropathogenic E. coli strains CECT 4484 and CECT 4485 | | | | |
|---|---|---|---|---|
| Source | Serotype | Main subunit Fimbria/PM kDA | Hemolysin | Adherence |
| strain CECT 4484 | Urine♀ 04:K- | P/17.5 | + | >100bact/epithelial cell |
| strain CECT 4485 | Urine♀ O5:K13 | 1/18.5 | + | >100bact/epithelial cell |

The bacteria are seeded in a liquid culture medium at 37º C until cultures are obtained at the beginning of the stationary phase (10⁸-10⁹ ufc/ml.)

The cultures are centrifuged and the obtained sediment is washed by means of centrifugation, for the purpose of eliminating the culture medium and the components that may exist in it.

The washed sediments containing the bacteria and resuspended in physiological saline solutions with a neutral pH are cold homogenized, in a shear homogenizer from 2 to 10 minutes, at a speed of 20,000 to 25,000 rpm.

The homogenates are centrifuged at a high speed at some 25,000-45,000 x g for 20-45 minutes and the sediment containing the bacteria is discarded.

The supernatant containing the fimbriae is recovered and several cold saline precipitations are carried out (which may be done with MgCl₂ or (NH₄)₂SO₄). The precipitation can be done for an ample period of time that goes from 2 hours to all night.

The precipitated material is recovered by means of centrifugation. It is reconstituted in aqueous physiological solutions of a neutral pH and afterwards it is dialyzed for the purpose of eliminating the residue of the salts used for precipitation, as well as the molecules smaller than 10,000 Da.

Afterwards, the fimbriae are treated with sodium deoxycholate 5 % at about 80º C for the purpose of eliminating the lipopolysaccharides (LPS.)

The material thus obtained (intact fimbriae) is subjected to chromatography in a gel filtration column containing Sephacryl S-200 (matrix for chromatography formed by a covalent bond of allyl-dextrane with N,N-methylenebisacrylamide, that gives rise to a rigid mesh in the form of spheres of 25-75 µm with pores that permit the inclusion of proteins of 1,000 to 80,000 Da) and subsequently the exclusion volume obtained herefrom is subjected to chromatography again in a Sepharose 4B column (matrix for chromatography formed by spheres of agarose of 45-165 µm with very little residue of groups with filler and pores that permit the inclusion of proteins of 6 x 10⁴ to 20 x 10⁶ Da), for the purpose of obtaining intact fimbriae free of too large substances, such as flagella or remains of membranes, etc., or of toxic substances with intermediate or low molecular weights, such as hemolysin, LPS etc. that could affect the destination of the product "Sephacryl S-200" and "Sepharose 4B" are the denominations of products being available in commerce under these names. Besides, with this, the elimination of the toxic substances that could otherwise be present in the product is also ensured.

The material contained in the inclusion volume of the Sepharose 4B column constitutes the product of the present invention, that has a molecular weight between 2x10⁵ Da and 20x10⁶ Da.

This product has practically no crossed reactivity between the type 1 and the type P fimbriae-adhesins isolated according to the present invention which allows the manufacture of immunogenes that possess each of the types of fimbria separately, as well as the types of fimbria-adhesins at the same concentration and that show a protective capacity in the experimental molding of urinary tract infection of mice.

Besides, the verification of the adherence capacity of the E. coli strains, associated to the fimbriae allows one to work with a material that induces immunoresponse against the adhesin fraction of the same cause of the fixation of E. coli to uroepithelial cells.

Once the fimbriae are obtained, the characteristics thereof have been studied, conducting determinations in the total sugar content, by the sugar reduction method (10) Park JT. Johnson MJ. A subminodetermination of glucose. J. Biol. Chem. 1949; 181:149-151; (11) Chaplin MF. Kennedy JF Carbohydrate analysis. A practical approach. IRL Press. Oxford. Washington, D.C. (1986), as well as the total protein content, by the Sutherland method ((12) Sutherland EW Cosi CF. Haynes R, Olson NS, Purification of the hyperglycemic-glycogenolitic factor from insulin and from gastric mucosa. J. Biol. Chem. 1949; 180:825-837) or by UV spectrophotometry.

Hence, the fimbriae obtained by the process of the present invention has shown to have 90-95% by weight of proteins and 1-3% by weight of sugar.

The degree of polymerization of the fimbria has also been determined by spectrophotometric measurement of the sample in the range of wave length that comprises the UV region (350-195 nm) (4.)

The SDS-PAGE study done on the grounds of Mc. Michael ((13) Mc Michael J. Ou. JT. Structure of common pili from E. coli. J. Bacteriol. 1979; 138:969-975) has emphasized the presence of fimbria fractions (that can be differentiated from the membrane proteins) due to their distinct electrophoretic mobility in the presence or absence of 2-β-mercaptoethanol and that can be perfectly differentiated between the different type of fimbriae.

The study of the detection and differentiation of carbohydrates by means of immunodetection with lecithins according to J. Montreuil et al. ((14) Montreuil J. Bouquelet S, Debray H. et al. Glycoproteins. Chapter 5 in MF Chaplin and JF Kennedy. Carbohydrate analysis. 1986. IRL Press. Oxford, Washington, D.C.) emphasizes the presence of carbohydrates associated to specific fractions of the fimbriae of the following form:
- Type 1 fimbriae, that show the presence of 5 different protein fraction with M.W. between 14 and 20 kDa, have a majority component (55%) of 17-18 kDa that is associated to carbohydrates;
- Type P fimbriae that show the presence of 5 protein fractions with M.W. between 14 and 20 kDa, have two majority components (35% and 30%) of 19-20 kDa and 15 kDa respectively, of which the first one is associated to carbohydrates.

The gel filtration chromatography by means of the SMART SYSTEM emphasizes the capacity of reorganization of the pilin subunits to form high molecular weight fimbriae, as well as for the subsequent purification thereof.

Analysis of the carbohydrates associated to fimbriae-adhesins by means of immunodetection with lecithins shows that said carbohydrates have mannose-mannose units α(1-3), α(1-6) or α(1-2), sialic acid α(2-6) or α(2-3) galactose, galactose β(1-3) n-acetyl galactosamine, galactose β(1-4) n-acetyl glucosamine.

On the other hand, the chromatographic analysis of fimbriae-adhesins, as well as of the protein subunits that comprise it shows that the fimbriae are kept in suspension in aqueous solutions and that the subunits are rapidly reorganized to form units of fimbriae and/or aggregates of the same of high molecular weight and that separate again in subunits of 14-20 kDa in the presence of alkaline solutions, anionic detergents, or 2-β-mercaptoethanol, and subjected to high temperatures (bath at 100º C.)

It is also noteworthy that, the purified type 1 as well as the purified type P fimbriae have the same characteristics as those that define them when they are associated to the bacterial body, hemagglutination and agglutination of S. cerevisiae (type 1) ((3) and (15) Domingue GJ, Roberts JA, Lauciria R. Pathogenic significance of O. fimbriated E. coli in urinary tract infections. J. Urology 1985; 133:983-989) and PPA technique, "receptor-specific" particle agglutination ((16) Svenson SB, Källenius G. Möllby R, Huttberg H. Winberg J. Rapid identification of P. fimbriated E. coli by a receptor-specific particle agglutination test. Infection 1982; 10:209-213.)

The intact fimbriae-adhesins obtained by the process of the present invention from E. coli strains with type 1 fimbriae (CECT 4485) and type P fimbriae (CECT 4484), isolated from urine of women with recurrent cystitis, have important uses as immunogenes and constitute the product used in this invention as the immunizator to obtain poly- or monoclonal antibodies.

The immunogenic capacity of the fimbriae-adhesins makes it possible to produce specific type 1 anti-fimbriae-adhesin and type P anti-fimbriae-adhesin antibodies, both poly and monoclonal ones, that on the other hand have practically no crossed reactivity. Said antibodies can be used as diagnostic tools to typify the type of uropathogenic E. coli fimbriae-adhesins. On the other hand, the use of intact fimbriae-adhesins as protective agents against UTI proves to be effective during active immunization with said components. The products that, specifically, have been used therapeutically in the prevention of urinary tract infections produced by uropathogenic E. coli are: (1) intact fimbriae in aqueous suspension, (2) fimbriae with coadjuvants, wither adsorbed to aluminum hydroxide gel, or bonded covalently to high molecular weight polysaccharides (such as for example pululan which is a linear glucose polymer (maltotriose) produced by Aureobosidium pullulans) and (3) in nanoparticles orally.

The experimental studies of induction of specific immune response against intact fimbriae proved effective in humoral immune response with production of specific antibodies, as well as the early induction of the natural cellular immune response by means of histopathological studies. The presence of inflammatory infiltrates with the accumulation of white blood cells (macrophages and lymphocytes) makes one think about the beginning specific immune cellular response subsequent to the urinary tract infection.

Likewise, immunization with type 1 and type P fimbriae prior to UTI in mice, causes significant differences between experimentally infected groups, depending on whether or not they have been previously immunized, therefore it can be concuded that this type of active immunization causes a specific protection in UTI caused by uropathogenic E. coli strains.

The specific polyclonal (anti-fimbriae) antibodies obtained in mice are capable of inhibiting the adhesion capacity of type 1 and type P fimbriae-adhesins.

The intact immunogenes (fimbriae) or immunogenes with adjuvants are administered in the form of aqueous suspensions (saline solution buffered with merthiolate 0.01 %) that have proven effective in a broad range of doses 0.1 µg-100 µg/kg. weight, administered in 1 to 4 doses, spaced in time with a minimum of 3o days (as long as more than one dose is administered.) The adjuvants normally used are aluminum hydroxide gel, calcium phosphate gel and pululan.

The immunogenes are normally administered subcutaneously and intraperitoneally.

At any rate, other ways of administration such as orally, in capsules or microparticles, as well as intra-urethrally in suspension form, cannot be excluded.

### EMBODIMENTS OF THE INVENTION

The present invention is additionally illustrated by means of the following Example, which does not aim to restrict the scope thereof.

### EXAMPLE

### Strains and characteristics of the same

Different E. coli strains were isolated from the urinary tract of women with recurrent cystitis, that were typified as to the existence of fimbriae by means of agglutination techniques in the presence or absence of mannose, adhesion capacity to human uroepithelial cells, characteristics of the present serotypes by means of specific immunoserums and production of hemolysin detectable in agar blood cultures. Finally, the following strains were selected:

| Strain | Serotype | Fimbria type | Adherence | Hemolysin |
|---|---|---|---|---|
| IAlP10 | 07:K1 | 1 and P | 89 bact/cell | - |
| IAlP2H | 06:K2 | 1 and P | >100 bact/cell | + |
| CECT 4485 | O5:K13 | 1 | >100 bact/cell | + |
| IAlOH | 083:K- | 1 | >100 bact/cell | - |
| CECT 4484 | O4:K- | P | >100 bact/cell | + |
| IAXl30 | 025:K13 | X | 57 bact/cell | + |

### Method of preparation and characterization

Uropathogenic E. coli strains were seeded in nutritive broth and were incubated all night at 37^{º} C. 1 ml. of these cultures (10⁸-10⁹ ufc/ml) was seeded in 500 ml. of nutritive broth, and incubation at 37^{º} C was carried out all night, without stirring. The cultures already grown were centrifuged at 10,000-20,000 x g for 20 minutes, three times, for the purpose of eliminating the culture medium, and the precipitate was resuspended in sterile saline solution (SS) (45 ml/500 ml culture.) The bacteria in SS were cold homogenized (4^{º} C) in a mechanical shear homogenizer, with 4 pulses of 1 minute at 18,750 rpm with intervals of 3 minutes between different pulses. (Virtishear-24 homogenizer with macro assembler and shears.) The homogenized material (bacteria and fimbriae separated from the bacteria) was cold centrifuged at 10,000 x g for 20 minutes. The supernatant containing the fimbriae that were purified by saline precipitation was recovered; carried out with MgCl₂ (20.3 g of MgCl₂.3H₂O/Liter of supernatant) cold. The precipitated material was recovered by centrifugation (20,000 x g, 30 minutes at 4^{º} C) which was resuspended in PBS O.02 M pH = 7.4. The saline precipitation was repeated 3 times. The material (fimbriae), resuspended in PBS after the last precipitation was dialzed in dialysis tubes, exhaustively against distilled water.

The fimbriae thus isolated were treated with sodium deoxycholate 5 % at a temperature of 80^{º} C for 15 minutes and were dialyzed again against distilled water.

The samples were passed through a Sephacryl S-200 HR column (XK26/100 Pharmacia) and were monitored in ISCO VA-5 equipment with a fraction retriever, Retriever II. Tris-HCl 50 mM pH=8 elution buffer was used, at a flow of 40 ml/h. The fractions corresponding to the elution volume were gathered and they were passed through a column of the same characteristics and in the same conditions, but with Sepharose 4B gel.

The material that is retrieved was the inclusion volume. Said material was exhaustively dialyzed against distilled water and constituted the base for all the subquent experiments.

The quantification of sugar (11) (sugar reduction method for quantification of monosaccharides by the neocuproine reagent) and of proteins (4) (spectrophotometric measurement at λ = 280 nm with bovine seroalbumin standards) with samples of fimbriae separated by heat and SDS allowed the study of the yield of the process to prepare fimbriae that was of 3-10 mg of fimbrial proteins per liter of culture and of 0.03-0.5 mg of monosaccharide sugar per liter of culture medium.

The treatment of the fimbriae based on the Mc Michael et al. method (13) allowed the evaluation of the protein components of said fimbriae (acidification of the sample to pH = 2, heating from 10 minutes at 100º C, alkalinization of the sample to pH = 10. Centrifugation and retrieval of the supernatant that was neutralized to pH = 7. Precipitations were done with ammonium sulfate and the precipitate which was resuspended in buffer sample Tris-HCl 0.5 M pH = 6.8, SDS 10%-with and without 2-β-mercaptoethanol. The samples were heated to 100º C for 2 or 10 minutes.

Electrophoresis in a discontinuous SDS-PAGE system at 12.5% was carried out based on the Laemmli method ((17) Laemmli UK. Cleavage of structural proteins during the asembly of the head of bacteriophage T₄. Nature 1970; 277:680-685.)

The results showed a main band corresponding to more than 95% of all the fimbrial proteins, of type 1 fimbriae proteins (19 kDa) as well as of type P fimbriae proteins ((18 kDa). with standards that differ slightly between the two types of fimbriae, when said samples were heated for 2 minutes.

When the samples were subjected to longer heating (10 minutes), the electrophoretic standard of both types of fimbriae differ notably. Hence, type 1 fimbriae show 5 different fractions with molecular weight between 21 and 13 kDa with two majority bands 33% and 31% of 19 to 15 kDa.

Type P fimbriae show 5 bands comprised between 20 and 14 kDa with a main component 53% of 18 kDa.

The detection and differentiation of glycoproteins associated to pilin bands (11) revealed by means of immuno-detection of lecithins by the method designed by Boehringer Mannheim (Glycan Detection Kit and Glycan Differentiation Kit), in fractions fixed to nitrocellulose membranes showed the presence of sugars associated to the pilin bands in both types of fimbriae. The lecithins used recognized terminal mannose residue α(1-3), α(1-6) or α(1-2), bonded to mannose, sialic acid bonded to α(2-3) galactose and galactose β(1-3) N-acetylgalactosamine. Said recognition may be, at the terminal level as well as at the middle level, given the previous treatment to which the sample is subjected.

Whether or not 2-β-mercaptoethanol (2 β BE) is present determines the modification in the mobility of the 19 kDa and 18 kDa fractions of the type 1 and type P fimbriae respectively. This indicates that the subunits that appear form part of the fimbriae (13.) Electrophotometric analysis of wave lengths comprised in the ultraviolet range show the presence of a relative maximum at 290 nm in fimbriae isolated directly from the preparation process and that disappear after treatment of the same with separating agents that characterize the integrity or degree of polymerization of the fimbriae.

Analysis by means of highresolution microchromatography by the SMART system (Pharmacia Biotech) with filtration microcolumns in Superdex 75 Pc 3.2 gel (30) according to the methodology proposed by the manufacturer shows the aggregating characteristic of the pilin subunits in aqueous media. Pilin subunits separated by acid-base shocks, 2-β-mercaptoethanol, SDS, urea and/or heat, rapidly group together again in an aqueous medium once the separating agents have been removed, retrieving the samples again in the exclusion volume of the microchromatography, indicating as other authors (13) point out the hydrophobic nature of these proteins.

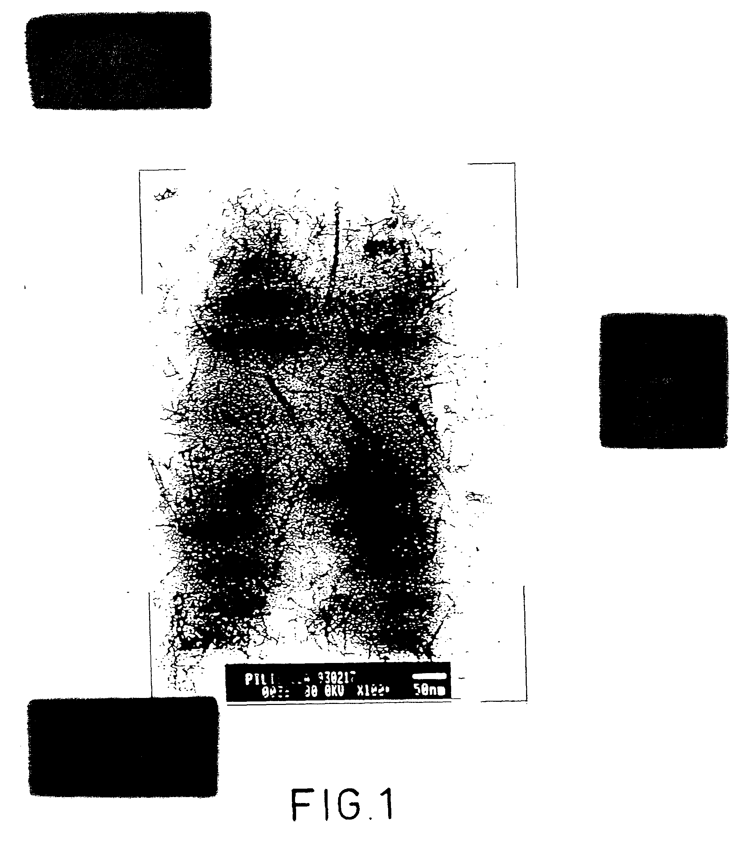

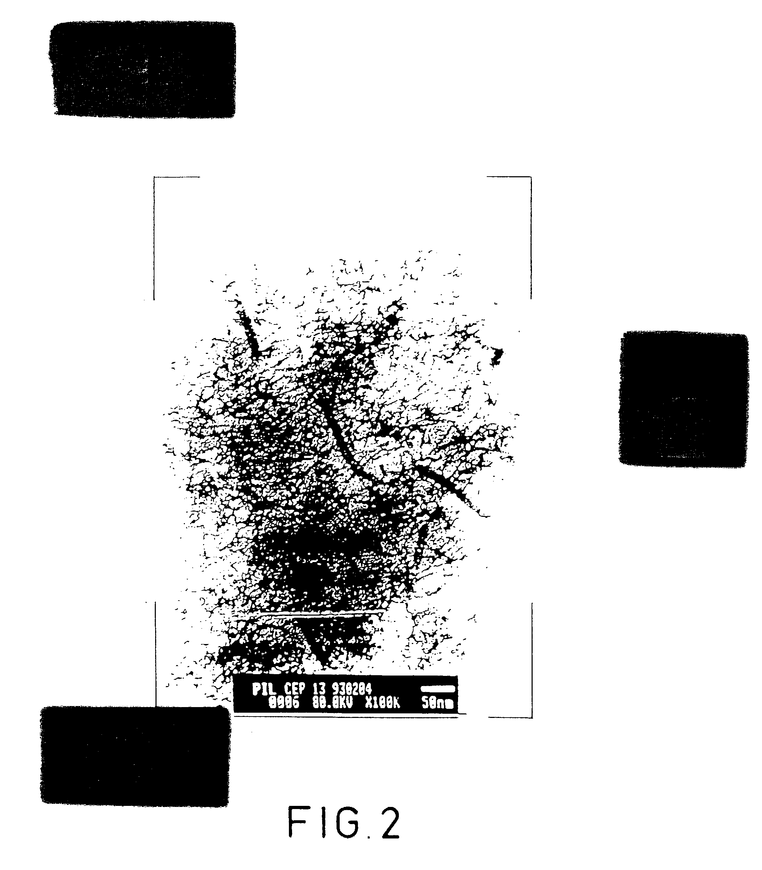

The electronic microscopic transmission studies showed the presence of fimbriae in the two E coli strains studied (CECT 4484 and CECT 4485.) The microphotographs of the purified fimbriae show a high degree of purity of the same (figures 1 and 2).

### Experimental study

For the purpose of studying a model that allows one to see the degree of protection that could be obtained by means of active immunization of animals with fimbriae, an experimental study with Balb/c mice was designed.

4 batches of 20 Balb/c female mice (each batch) were used, as is expressed in the protocol.

The evaluation of the study was carried out: (I) by DOT-ELISA techniques, just as it is described in Bjerrum et al. ((18) Gordon J. Billing P. Dot-Immunoblotting. General principles and procedures. In Bjerrun OJ and Heegaard NH. Eds. Handbook of Immunoblotting of proteins. Vol. I. CRC Press 1988.PP: 27-30) to measure the level of specific antibodies (titer) from serum and urine; (II) by means of isolation (6) and identification API System) of bacteria from necroscopic pieces. The typification of the type of fimbriae of the isolated E. coli bacteria was done by means of SDS-PAGE of the fimbriae obtained from each isolation of E. coli; (III) by means of histopathological studied ((19) Bancroft JB, Stevens A (Ed.) Theory and Practice of Histological Techniques. 1982; 2nd. Edition. Churchill and Livingstone. Edimburgh) of the necroscopic pieces (kidneys and bladder.)

### Protocol of the Experimental Study:

| BATCH N^{º} | INDIVIDUALS | BREED | DEFINITION OF BATCH | CHARACTERISTICS | SAMPLES STUDIED |
|---|---|---|---|---|---|
| 1 | 10 FEMALES | BALB/c | CONTROL OF HEALTHY ONES | HEALTHY MICE, NOT IMMUNIZED, NOT INFECTED | SERUM, BLADDER AND KIDNEYS |
| 2 | 20 FEMALES | BALB/c | INFECTION WITH E. COLI IA l 13 H | HEALTHY MICE, NOT IMMUNIZED, INFECTED WITH E. COLI CECT 4485 | POST-INFECTION SERUM, PRE- AND POST-INFECTION URINE BLADDER AND KIDNEYS |
| 3 | 20 FEMALES | BALB/c | INFECTION WITH E. COLI IA P O H | HEALTHY MICE, NOT IMMUNIZED, INFECTED WITH E. COLI CECT 4484 | POST-INFECTION SERUM, PRE- AND POST-INFECTION URINE BLADDER AND KIDNEYS |
| 4 | 20 FEMALES | BALB/c | IMMUNIZATION AND INFECTION | HEALTHY MICE IMMUNIZED WITH FIMBRIA 1 § FIMBRIA P AND THEN INFECTED WITH BOTH STRAINS (1:1) | POSTIMMUNIZATION AND POSTINFECTION SERUM, PRE- AND POSTINFECTION URINE, BLADDER AND KIDNEYS |
| 7 | 10 FEMALES | BALB/c | MICE IMMUNIZED NOT INFECTED | HEALTHY MICE IMMUNIZED WITH FIMBRIA 1 § FIMBRIA P | PRE- AND POSTIMMUNIZATION URINE BLOOD DAYS 0, 15 AND 30, BLADDER AND KIDNEYS |

### INFECTION MODEL

* Nº Batches : 4
* Size and Identification Batches :
SAMPLE BATCH HEALTHY : Batch Nº 1. Not infected/
not immunized. 10 animals
CONTROL BATCH INFECTED : Batch Nº 2. Infected with
E. coli CECT 4485 20
animals
CONTROL BATCH INFECTED : Batch Nº 3. Infected with
E. coli CECT 4484 20
animals
* Catheters : Sterile stainless steel 0.5
mm diameter
* Anesthesia : 0.15 - 0.20 ml. i.m. admini-
stration "Zoletil 20" (Tiletha
mine + Zolazepam) 4 mg/ml
* Inoculum : E. coli CECT 4485 § CECT 4485
Bacto nutrient broth- -CULTIVATED
24 h. 37º
CENTRIFUGATION
10000 rpm/20'/4º C
SEDIMENT SUPERNATANT
sterile ssf suspension 25 ml
RECONSTITUTION
CENTRIFUGATION
10000 rpm/29'/4ºC
SEDIMENT SUPERNATANT
SUSPENSION SSF AND
ADJUST TO 10^9 ufc/ml
INOCULUM

Viability by means of seeding 1 drop of Mac Conkey agar

### IMMUNIZATION MODEL

ANIMALS : BALB-c female mice CAGES : 5 animals
Age : 8 - 10 weeks
Average weight : 20 g.

IDENTIFICATION : Digitalized : Batch/Cage/Nº Mouse

Example : CODE B2C3M5
- Batch Nº 2
- Cage Nº 3
- Mouse Nº 5

IMMUNIZATION MODEL :
* Nº Batches : 2

* Size and Identification Batches :
CONTROL BATCH - BATCH Nº 7 : Mice immunized with
Fimbriae 1 and P
10 animals
TEST BATCH - BATCH Nº 4 : Mice immunized with
Fimbriae 1 and P and
infected with serotypes
CECT 4485 and CECT 4484
20 animals

* Way of Administration : SC in complete Freund
adjuvant

* Administration dose : 25 mcg type 1 Fimbriae
4
weekly
25 mcg type P Fimbriae doses

### Animal Immunization-Infection Model Controls

- * Urinary control :: Discard mice with positive urine culture from samples of spontaneous urination. Urine culture medium : Mac Conkey and Nutrient agar
- * Infection :: Catheterism through the meatus urinarius and medium intravesical injection of 0.1 ml of a suspension of bacteria adjusted to 10⁹ ufc/ml
- * Autopsy :: Killing : 72 h post-infection vesical
Bloodletting: Puncture of the inside angle of the eye with Pasteur pipettes in anesthetized animal and pouring into Eppendorf tubes for retraction of the clot and obtainment of serum.
Samples : Medium incision from the pelvis to the base of the neck, extended upon the paws, after disinfecting the skin with ethanol. Additional medium incision to open so as to have access to the abdominal cavity.
Obtainment of sterile urine: Puncture bladder with a needle with a 0.5 mm diameter and suction in a 1 ml. syringe for subsequent culture thereof and identification of antibodies. Obtainment of bladders: After removing urine, the bladder was sectioned and the bladder was removed at its base, transferring it to a vial with buffered formol tor the histopathological study. Obtainment of kidneys: Dissection and removal of both kidneys and sagittal cut of the same at the renal pelvis level in two equal parts. Microbiological analysis by smear of the surface of the cut section on plates with Mac Conkey agar and incubation 37º C/24 h. Histopathological analysis

### Overall results of the Study of Experimental Infection in the Different Batches of Mice

| STRAIN AND BATCH N^{º} | TOTAL INOCULATED ANIMALS | TOTAL IMMUNIZED ANIMALS | TOTAL INFECTED ANIMALS (%) |
|---|---|---|---|
| CECT 4485 (BATCH 2) | 20 | 0 | 16 (80) |
| CECT 4484 (BATCH 3) | 20 | 0 | 8 (40) |
| CECT 4485 (BATCH 4) | 20 | 20 (FIMBRIA l) | 7 (35) |
| CECT 4484 (BATCH 4) | 20 | 20 (FIMBRIA P) | 0 (0) |

The histopathological studies showed that inoculation of female mice through the urethra produced pyelitis, urethritis or acute cystitis.

In all cases, the inflammatory phenomena are limited to the transitional epithelium of the calyxes and the connective tissue subjacent to the same and to a lesser degree, segmentary inflammatory phenomena of the proximal portion of the urethra are observed. There are no foci of infection in the parenchyma.

### Study of the immune response of the mouse to immunization with fimbrial antigens.

The humoral immune response was studied by measuring the titer and characteristics of the specific antibodies by means of the DOT-ELISA technique, as described by Gordon et al. (18) ELISA based on Muñoz et al. ((20) Muñoz C, Nieto A, Gaya A, Martinez J. Vives J. New experimental criteria for optimization of solid-phase antigen concentration and stability in ELISA. J Immunol. Methods 1986; 94: 137-144) and SDS-PAGE Immunoblotting ((21) Towbin H, Staehilin I, Gordon J. Electrophoretic transfer of proteins from polyacrylamide gels to nitrocellulose sheet: procedure and some applications. Proc. Natl Acad. Sci. USA 1979; 76:4350-4354)

The study of crossed reactivity between type 1 and type fimbriae was done by ELISA inhibition, according to the method described by Martinez et al. ((22) Martinez J. Nieto A, Vives J. Torres JM. Application of ELISA inhibition to Aspergillus antigen standardization for immunodiagnosis. J. Med Vet Micol 1985; 23:317-320.)

The results showed a clear increase of the titer of specific antibodies that were from negative values in all individuals, before being immunized, up to titers (DOT-ELISA) of more than 1,280 and absorbance difference values (ELISA) of 1.5 at serum dilutions of 1/1,000 after three immunization with semi-monthly intervals.

Immunoblotting showed the specificity of the reaction of the antibodies themselves, against fractions of 17 kDa (type 1) and of 19 kDa (type P), corresponding to the fundamental components of the fimbriae.

The study of crossed reactivity by ELISA did not show any significant crossed reactivity.

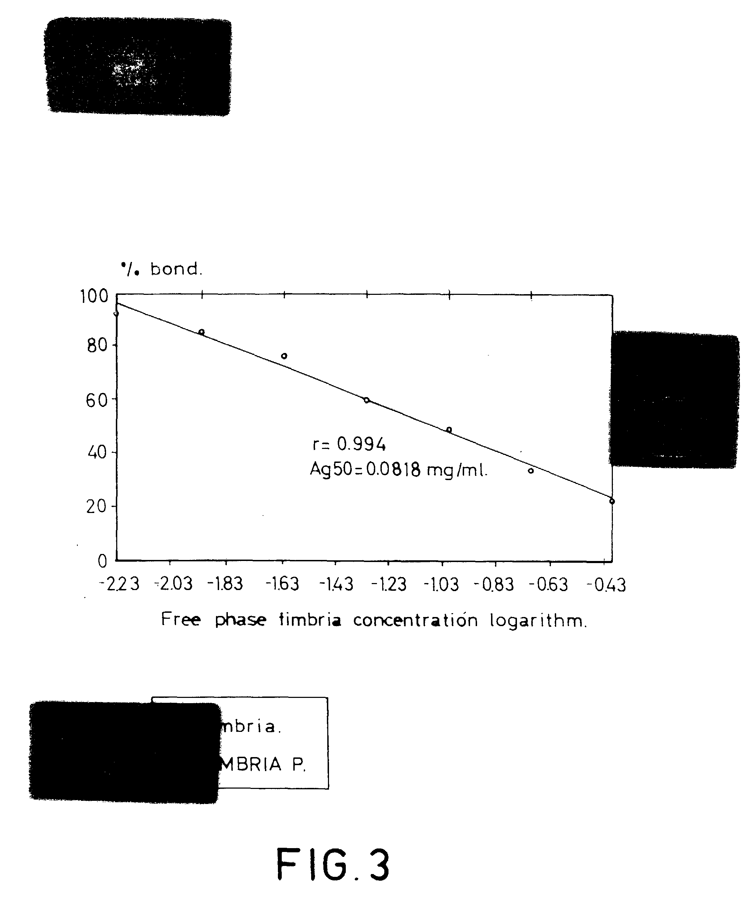

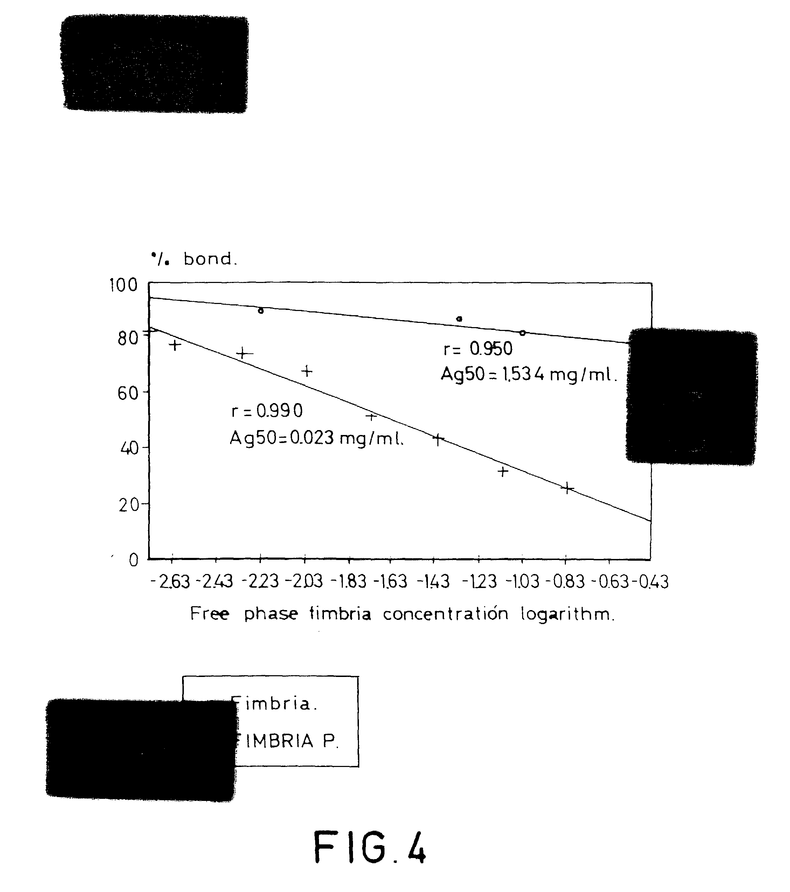

As can be seen in the graphs of Figures 3 and 4, between type 1 fimbriae (solid phase) and type P fimbriae (inhibitory phase), no crossed reactivity was observed. Between type P fimbriae (solid phase) and type 1 fimbriae (inhibitory phase) very scarce crossed reactivity that was less than 2% and with differences of Ag₅₀ of both systems higher than 5 x 10³ was detected.

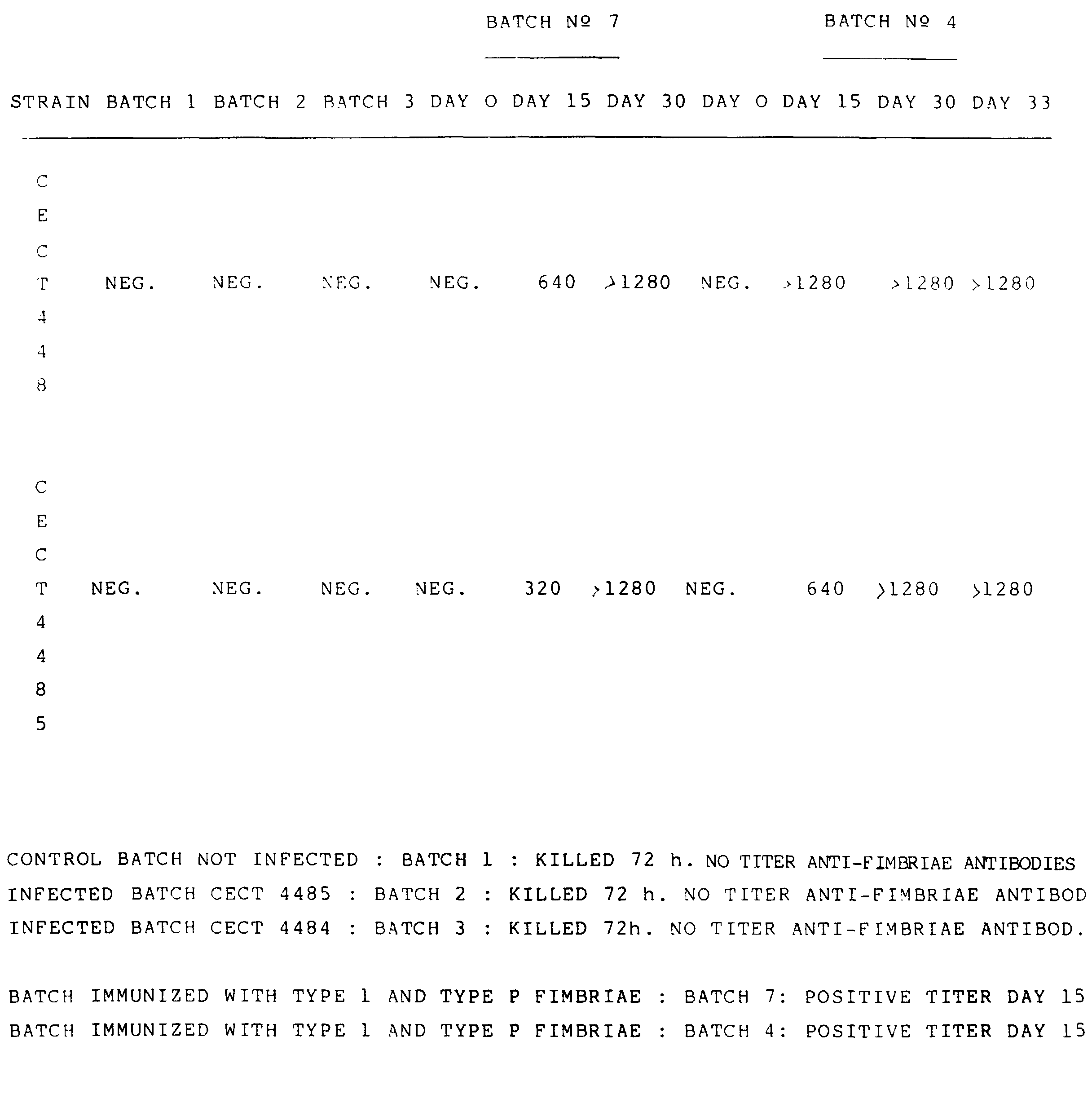

### Levels of Specific IgA in Urine of Mice of the Experimental Study by DOT-ELISA

| STRAIN | BATCH 1 | BATCH 2 | BATCH 3 | BATCH N^{º} 4 | | BATCH N^{º} 7 | |
|---|---|---|---|---|---|---|---|
| | | | | PREINFECTION | POSTINFECTION | PREIMMUNIZATION | POSTIMMUNIZATION |
| CECT 4484 | NEG. | NEG. | NEG. | NEGATIVE | NEGATIVE | NEGATIVE | NEGATIVE |
| CECT 4485 | NEG. | NEG. | NEG. | NEGATIVE | NEGATIVE | NEGATIVE | NEGATIVE |

The statistical analysis shown in the previous table (titled "Overall results of the study of experimental infection in the different batches of mice") showed the existence of significant differences between the control batch not infected, the infected batches and the ones immunized prior to infection.

### Toxicity test

Abnormal toxicity tests were carried out on guinea pigs (intraperitoneal administration), local tolerance subcutaneously on mice and abnormal toxicity in mice (intraperitoneal administration), according to standard protocols carried out by "Centro de Investigación y Desarrollo Aplicado, S.A.L." (Research and Applied Development Center, Ltd.) (homologated.)

The results did not show any element that came into conflict with the rules of European Pharmacopeia, so as to include it as an unacceptable product.

## Claims

1. Type 1 fimbriae-adhesin isolated from intact fimbriae of *E*. *coli* strain CECT 4485 and Type P fimbriae-adhesin isolated from *E. coli* strain CECT 4484 each fimbriae-adhesin having a molecular weight comprised between 2x10⁵ and 2x10⁷ and being comprised of 90-95% by weight of protein and 1-3% by weight of sugar, each of the fimbriae-adhesins further comprising 5 different protein fractions including a majority component that is linked to carbohydrates, said carbohydrates containing α(1-3), α(1-6), or α(1-2) mannose-mannose, α(2-6) or α(2-3)galactose sialic acid, β(1-3) galactose n-acetyl galactosamine, β(1-4)galactose n-acetyl glucosamine, the 5 protein fractions of Type 1 fimbriae-adhesin having molecular weights comprised between 14-20 kDa with a main component of a molecular weight of 17-18 kDa, and the 5 protein fractions of Type P fimbriae-adhesin having molecular weights comprised between 14 and 20 kDa with a first majority component of a molecular weight of 19-20 kDa and a second majority component having a molecular weight of 15 kDa which is not linked to carbohydrates.

2. A process for preparing any of the fimbriae-adhesins of claim 1, the process comprising the following steps:
a first step of seeding *E. coli* bacteria selected from one of the strains CECT 4484 and CECT 4485 in a liquid culture medium at about 37°C until obtaining cultures in an initial stage of a stationary phase (10⁸-10⁹ ucf/ml);
a second step of centrifuging the cultures resulting from the first step whereby a first sediment is obtained, and washing the first sediment to obtain a washed sediment;
a third step of resuspending the washed sediment in a physiological saline solution having a neutral pH to obtain a suspension, and cold homogenizing the suspension in a shear homogenizer for 2 to 10 minutes, at a speed of 10,000 to 25,000 rpm, to obtain a homogenate;
a fourth step of centrifuging the homogenates obtained by the third step at 25,000 - 45,000 x g for 20-45 minutes until obtaining a second sediment containing bacteria and a supernatant liquid containing fimbriae, discarding the second sediment and recovering the supernatant liquid;
a fifth step of subjecting the supernatant liquid containing the fimbriae to several cold saline precipitations for 2 and 15 hours until obtaining a precipitate, recovering the precipitate by centrifugation to obtain a centrifugated precipitate;
a sixth step of reconstituting the centrifugated precipitate in an aqueous physiological solution of a neutral pH to obtain a precipitate solution and dialyzing the precipitate solution to obtain a dialyzed solution containng the fimbriae;
a seventh step of treating the dialyzed solution with sodium doexoxycholate 5% at about 80°C to obtain a first fimbriae containing product;
an eight step of subjecting said first product to chromatography in a gel filtration column containing SEPHACRYL S-200 until obtaining a chromatographed product, and subjecting the chromatographed product (exclusion volume) to further chromatography in a column containing SEPHAROSE 4B (inclusion volume), to obtain the fimbriae-adhesins.

3. Use of the fimbriae-adhesins defined in claim 1, in the manufacture of medicines for treating urinary tract infections produced by fimbriated *E. coli.*

4. Use of the fimbriae-adhesins defined in claim 1, in the manufacture of vaccines for preventing urinary tract infections produced by fimbriated *E. coli.*

5. Use of the fimbriae-adhesins defined in claim 1, in immunization, for production of anti-fimbria-adhesin antibodies for the diagnosis and typification of uropathogenic *E. coli.*

6. Fimbriae-adhesins according to claim 1, wherein the majority component among the five protein fractions of the Type 1 fimbriae-adhesins is present in a proportion of 55%.

7. Fimbriae-adhesins according to claim 1, wherein the majority component among the five protein fractions of the Type P fimbriae-adhesins is present in a proportion of 35%.

8. Fimbriae-adhesins according to claim 1, wherein said secondary majority component is present in a proportion of 30%.

## Patentansprüche

1. Typ 1-Fimbrien-Adhäsin, isoliert aus intakten Fimbrien des E. coli-Stamms CECT 4485, und Typ P-Fimbrien-Adhäsin, isoliert aus dem E. coli-Stamm CECT 4484, wobei jedes Fimbrien-Adhäsin ein Molekulargewicht zwischen 2x10⁵ und 2x10⁷ aufweist und 90-95 Gew.-% Protein und 1-3 Gew.-% Zucker umfaßt, wobei jedes der Fimbrien-Adhäsine außerdem 5 verschiedene Protein-Fraktionen umfaßt einschließlich einer Mehrheitskomponente, die mit Kohlenhydraten verbunden ist, wobei die Kohlenhydrate α (1-3), α (1-6) oder α (1-2) Mannose-mannose, α(2-6) oder α(2-3) Galactose-sialinsäure, β(1-3) Galactose-N-acetylgalactosamin, β(1-4) Galactose-N-acetylglucosamin enthalten, die 5 Proteinfraktionen vom Typ 1-Fimbrien-Adhäsin Molekulargewichte zwischen 14-20 kDa mit einer Hauptkomponente mit einem Molekulargewicht von 17 - 18 kDa aufweisen, und die 5 Proteinfraktionen vom Typ P-Fimbrien-Adhäsin Molekulargewichte zwischen 14 und 20 kDa mit einer ersten Mehrheitskomponente mit einem Molekulargewicht von 19- 20 kDa und einer zweiten Mehrheitskomponente mit einem Molekulargewicht von 15 kDa, welche nicht mit Kohlenhydraten verbunden ist, aufweisen.

2. Verfahren zum Herstellen eines beliebigen der Fimbrien-Adhäsine nach Anspruch 1, wobei das Verfahren die folgenden Schritte umfaßt:
einen ersten Schritt des Einimpfens von E. coli-Bakterien, ausgewählt aus einem der Stämme CECT 4484 und CECT 4485, in ein flüssiges Kulturmedium bei ungefähr 37°C, bis zum Erhalt von Kulturen in einem Anfangsstadium einer stationären Phase (10⁸ - 10⁹ Kolonie-bildende Einheiten/ml (10⁸ - 10⁹ ufc/ml));
einen zweiten Schritt des Zentrifugierens der Kulturen, die sich aus dem ersten Schritt ergeben, wodurch ein erstes Sediment erhalten wird, und des Waschens des ersten Sediments, um ein gewaschenes Sediment zu erhalten;
einen dritten Schritt des Resuspendierens des gewaschenen Sediments in einer physiologischen Kochsalzlösung mit einem neutralen pH, um eine Suspension zu erhalten, und des kalten Homogenisierens der Suspension in einem Scherhomogenisator während 2 bis 10 Minuten mit einer Geschwindigkeit von 10000 bis 25000 U/min, um ein Homogenat zu erhalten;
einen vierten Schritt des Zentrifugierens der Homogenate, die durch den dritten Schritt erhalten wurden, bei 25000 bis 45000 x g während 20-45 Minuten bis zum Erhalt eines zweiten Sediments, das Bakterien enthält, und einer Überstandsflüssigkeit, die Fimbrien enthält, des Verwerfens des zweiten Sediments und des Gewinnens der Überstandsflüssigkeit;
einen fünften Schritt des Unterziehens der Überstandsflüssigkeit, welche die Fimbrien enthält, mehreren kalten Kochsalzlösungsfällungen während 2 und 15 Stunden, bis zum Erhalt eines Niederschlags, und des Gewinnens des Niederschlags durch Zentrifugation, um einen zentrifugierten Niederschlag zu erhalten;
einen sechsten Schritt des Wiederauflösens des zentrifugierten Niederschlags in einer wäßrigen physiologischen Lösung mit neutralem pH, um eine Niederschlagslösung zu erhalten, und des Dialysierens der Niederschlagslösung, um eine dialysierte Lösung, welche die Fimbrien enthält, zu erhalten;
einen siebten Schritt des Behandelns der dialysierten Lösung mit 5% Natriumdesoxycholat bei ungefähr 80°C, um ein erstes Fimbrien enthaltendes Produkt zu erhalten;
einen achten Schritt des Unterziehens des ersten Produkts einer Chromatographie in einer Gelfiltrationssäule, die SEPHACRYL S-200 enthält, bis zum Erhalt eines chromatographierten Produkts, und des Unterziehens des chromatographierten Produkts (Ausschlußvolumen) einer weiteren Chromatographie in einer Säule, die SEPHAROSE 4B enthält (Einschlußvolumen), um die Fimbrien-Adhäsine zu erhalten.

3. Verwendung der Fimbrien-Adhäsine, die in Anspruch 1 definiert sind, bei der Herstellung von Arzneimitteln zur Behandlung von Harntrakt-lnfektionen, die durch fimbrientragendes E. coli hervorgerufen werden.

4. Verwendung der Fimbrien-Adhäsine, die in Anspruch 1 definiert sind, bei der Herstellung von Impfstoffen zum Verhindern von Harntrakt-lnfektionen, die durch fimbrientragendes E. coli hervorgerufen werden.

5. Verwendung der Fimbrien-Adhäsine, die in Anspruch 1 definiert sind, bei der Immunisierung zur Herstellung von Anti-Fimbrien-Adhäsin-Antikörpem für die Diagnose und Typisierung von uropathogenen E. coli.

6. Fimbrien-Adhäsine nach Anspruch 1, worin die Mehrheitskomponente unter den fünf Proteinfraktionen der Typ 1-Fimbrien-Adhäsine in einem Anteil von 55% vorhanden ist.

7. Fimbrien-Adhäsine nach Anspruch 1, worin die Mehrheitskomponente unter den fünf Proteinfraktionen der Typ P-Fimbrien-Adhäsine in einem Anteil von 35% vorhanden ist.

8. Fimbrien-Adhäsine nach Anspruch 1, worin die zweite Mehrheitskomponente in einem Anteil von 30% vorhanden ist.

## Revendications

1. Adhésine fimbriale de type 1 isolée à partir de fimbriae intactes de la souche *E. coli* CECT 4485 et adhésine fimbriale de type P isolée de la souche *E. coli* CECT 4484, chaque adhésine fimbriale ayant un poids moléculaire compris entre 2 x 10⁵ et 2 x 10⁷ et étant constituée de 90 à 95 % en poids de protéine et de 1 à 3 % en poids de sucre, chaque adhésine fimbriale comprenant en plus 5 fractions protéiniques différentes contenant un composant majoritaire qui est lié à des hydrates de carbones, lesdits hydrates de carbone contenant du α(1-3), α(1-6) ou α(1-2) mannose-mannose, de l'acide α(2-6) ou α(2-3) galactose sialique, de la β(1-3) galactose n-acétyl galactosamine, de la β(1-4) galactose glucosamine, les 5 fractions protéiniques de la adhésine fimbriale de type 1 ayant des poids moléculaires compris entre 14 et 20 kDa avec un composant principal ayant un poids moléculaire de 17 à 18 kDa, et les 5 fractions protéiniques de la adhésine fimbriale de type P ayant des poids moléculaires compris entre 14 et 20 kDa avec un premier composant majoritaire ayant un poids moléculaire de 19 à 20 kDa et un deuxième composant majoritaire ayant un poids moléculaire de 15 kDa qui n'est pas lié à des hydrates de carbone.

2. Procédé de préparation de l'une quelconque des adhésines fimbriales de la revendication 1, ledit procédé comprenant les étapes suivantes :
une première étape consistant à ensemencer des bactéries *E. coli* choisies parmi l'une des souches CECT 4484 et CECT 4485 dans un milieu de culture liquide à environ 37 °C jusqu'à obtenir des cultures dans un stade initial d'une phase stationnaire (10⁸ à 10⁹ ufc/ml) ;
une deuxième étape consistant à centrifuger les cultures résultant de la première étape afin d'obtenir un premier culot de centrifugation, et à laver le premier culot de centrifugation afin d'obtenir un culot de centrifugation lavé ;
une troisième étape consistant à remettre le culot de centrifugation lavé en suspension dans une solution saline physiologique ayant un pH neutre afin d'obtenir une suspension, et à homogénéiser à froid la suspension dans un homogénéisateur à gradient de cisaillement pendant 2 à 10 minutes, à une vitesse de 10 000 à 25 000 tours par minute, afin d'obtenir un homogénéisat ;
une quatrième étape consistant à centrifuger l'homogénéisat obtenu dans la troisième étape entre 25 000 et 45 000 x g pendant 20 à 45 minutes jusqu'à obtenir un deuxième culot de centrifugation contenant des bactéries et un liquide surnageant contenant des fimbriae, à jeter le deuxième culot de centrifugation et à récupérer le liquide surnageant ;
une cinquième étape consistant à soumettre le liquide surnageant contenant les fimbriae à plusieurs précipitations à froid en milieu salin pendant 2 à 15 heures jusqu'à obtenir un précipité, et à récupérer le précipité par centrifugation afin d'obtenir un précipité centrifugé ;
une sixième étape consistant à reconstituer le précipité centrifugé dans une solution physiologique aqueuse ayant un pH neutre afin d'obtenir une solution de précipité, et à dialyser la solution de précipité afin d'obtenir une solution dialysée contenant les fimbriae ;
une septième étape consistant à traiter la solution dialysée avec du désoxycholate de sodium à 5 % à environ 80 °C afin d'obtenir un premier produit contenant des fimbriae ;
une huitième étape consistant à soumettre ledit premier produit à une chromatographie à perméation de gel sur une colonne contenant du SEPHACRYL S-200 jusqu'à obtenir un produit chromatographié, et à soumettre le produit chromatographié (volume d'exclusion) à une autre chromatographie dans une colonne contenant du SEPHAROSE 4B (volume d'inclusion), afin d'obtenir les adhésines fimbriales.

3. Utilisation des adhésines fimbriales définies dans la revendication 1 dans la fabrication de médicaments destinés au traitement d'infections des voies urinaires provoquées par des *E. coli* pourvues de fimbriae.

4. Utilisation des adhésines fimbriales définies dans la revendication 1 dans la fabrication de vaccins destinés à la prévention d'infections des voies urinaires provoquées par des *E. coli* pourvues de fimbriae.

5. Utilisation des adhésines fimbriales définies dans la revendication 1, en immunisation, pour la production d'anticorps anti-adhésines fimbriales destinés au diagnostic et à la typification des souches *E. coli* uropathogènes.

6. Adhésines fimbriales selon la revendication 1, dans lesquelles le composant majoritaire dans les cinq fractions protéiniques des adhésines fimbriales de type 1 1 est présent dans une proportion de 55 %.

7. Adhésines fimbriales selon la revendication 1, dans lesquelles le composant majoritaire dans les cinq fractions protéiniques des adhésines fimbriales de type P est présent dans une proportion de 35 %.

8. Adhésines fimbriales selon la revendication 1, dans lesquelles ledit deuxième composant majoritaire est présent dans une proportion de 30 %.
